# EUROPEAN PATENT SPECIFICATION

(11) **EP 0 572 986 B1**
(45) Date of publication and mention of the grant of the patent: **28.08.1996**
(21) Application number: 93108811.6
(22) Date of filing: 01.06.1993
(51) Int. Cl.: C07C 219/14, C07C 229/60, C12Q 1/46, C12Q 1/28

(54) **Assay for serum cholinesterase**
Bestimmungsverfahren für Serumcholinesterase
Essai pour la cholinesterase de sérum

(30) Priority: 01.06.1992 US 891897
(43) Date of publication of application: 08.12.1993
(73) Proprietor: Johnson & Johnson Clinical Diagnostics, Inc., Rochester New York 14650 (US)
(72) Inventor: Hasselberg, Stephen Carl, Eastman Kodak Company, Rochester, New York 14650-2201 (US); Babb, Bruce Edward, Eastman Kodak Company, Rochester, New York 14650-2201 (US); Engebrecht, Ronald Henry, Eastman Kodak Company, Rochester, New York 14650-2201 (US)
(74) Representative: Mercer, Christopher Paul

(56) References cited:
- EP-A- 0 155 825
- EP-A- 0 160 980
- EP-A- 0 241 915
- US-A- 4 271 310
- US-A- 4 357 363

## Description

The present invention relates to clinical chemistry. In particular, it relates to an element and a method for determining cholinesterase activity and to disubstituted benzoylcholine derivatives for use as substrates in the element.

Cholinesterase (specifically serum cholinesterase or pseudocholinesterase) is a serum enzyme useful in the diagnosis of liver diseases, pesticide poisoning, and for identifying individuals unable to metabolize the muscle relaxant suxamethonium (succinyldicholine).

Various methods for determining the activity of cholinesterase are known, and all of these known methods use choline esters as substrate (see for example EP-A0 241 915, EP-A 0 160 980). Cholinesterase hydrolyzes the substrate generating choline and the corresponding acid from the ester.

The most commonly used substrates, butyrylthiocholine, propionylthiocholine, and benzoylcholine, have very high rates of reaction with cholinesterase. When the hydrolyzing action of cholinesterase on the substrate proceeds too quickly, it is very difficult to trace the rate of reaction exactly by manual or mechanical operation. It is thus difficult to determine the amount of cholinesterase present with any degree of accuracy. Thus, the commonly used substrates require dilution of the test specimen, making the results prone to imprecision. The reasons for imprecision upon dilution are discussed in commonly owned U.S. Serial No. 763,383.

Moreover, these substrates have high rates of alkaline (non-enzymatic) hydrolysis (hydrolysis that occurs spontaneously under basic conditions), requiring that blank readings be taken. Because alkaline hydrolysis is a strong function of pH, the test is usually done at near neutral pH, which is suboptimal for the enzyme. Optimal pH is generally regarded to be between 8 and 8.5.

Further, serum cholinesterase activity varies considerably among individuals. Because of this, together with the aforementioned high rate of hydrolysis of the substrates, many analytical systems do not have a dynamic range broad enough to accommodate the range of activity normally found in patients. To solve this problem, further dilutions must be made of high activity samples.

What is desired, then, is a substrate for determining cholinesterase activity having a conveniently low rate of cholinesterase catalyzed hydrolysis, stability to alkaline hydrolysis, a broad dynamic range, and a high degree of accuracy. It is also desirable that the test operate at or near the pH optimum range of 8-8.5.

Accordingly, the present invention provides a substrate with a low rate of cholinesterase-catalyzed hydrolysis and a high degree of accuracy. Briefly summarized, according to one aspect of the invention, there is provided a substrate of the formula: 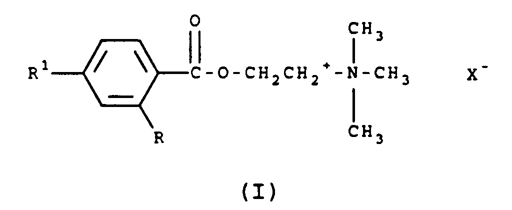
wherein R and R¹ each independently represents alkyl, alkoxy, amino (-NR²R³), wherein R² and R³ each independently represents hydrogen, methyl, ethyl, or propyl, and X⁻ is an anion.

In another aspect of the invention, there is provided a multilayer analytical element for the determination of serum cholinesterase comprising a support having thereon, in order from said support and in fluid contact, at least one reagent layer and a spreading layer, and containing in said reagent layer:
a) a substrate for serum cholinesterase as defined above;
b) choline oxidase;
c) peroxidase;
d) a color-developing reagent; and
e) a buffer having a pH of 7 to 9.

A method for determining serum cholinesterase is also provided.

The enzymatic rate of hydrolysis of the above-defined substrates is sufficiently low so that the serum to be tested may be undiluted. These substrates are also more stable to alkaline hydrolysis (spontaneous hydrolysis occurring at basic pH) than o-toluoylcholine and other commonly used substrates mentioned above. There is also less substrate inhibition with the present compounds than with other substrates like 2,3-dimethoxybenzoylcholine.

It was unexpected that the 1,4- di-substituted arylcholines of the invention would provide these advantages, especially since mono- and other di-substituted compounds (for example, o-toluoylcholine and 2,6-dimethyl benzoylcholine) did not work as well.

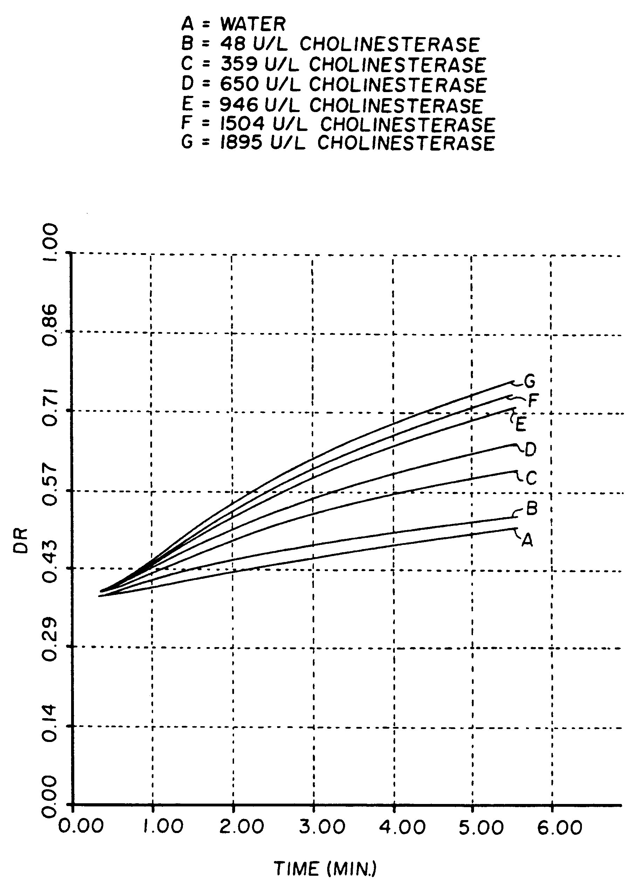

Figure 1 is a graph of kinetic traces, reflectance density versus time, for water and six fluids containing varying amounts of cholinesterase. The assay was carried out using the element of the invention in an EKTACHEM™ 700 clinical chemistry analyzer. The graph shows that the slope of the kinetic trace increases with increasing cholinesterase activity.

As used herein, the term "fluid contact" and similar terms refer to zones or layers of an element which are associated with one another in a manner such that, under conditions of use, a fluid, whether liquid or gaseous, can pass in the element between these layers or zones. Such fluid contact therefore refers to the capability of the element to permit passage of at least some components of a fluid sample between zones or layers of the element which are said to be in "fluid contact". Zones which are in fluid contact can be contiguous, but they also may be separated by intervening zones or layers. Such intervening zones or layers however will also be in fluid contact in this case and will not prevent the passage of fluid between the fluid contacting layers or zones.

The term "dry" as used herein to describe analytical methods refers to analytical methods and techniques that are carried out using chemical reagents contained in various "dry-to-the-touch" test elements such as "dip and read" test strips, multilayer test elements and the like. "Dry" methods require no liquid for reconstitution or analysis.

The term "dry coverage" as used herein indicates that the coating coverage is determined as "dry weight" after normal coating and drying processes.

The term "porous" as used herein means being full of pores such that a fluid can be absorbed by capillary action and can pass to other layers in fluid contact with the porous layer.

The term "reflective" as used herein means that incident light applied to the element would not be transmitted through the element but would be reflected back to a photodetector where the reflected density can be measured.

The present invention relates to the preparation and use of a family of cholinesterase substrates having a lower rate of cholinesterase catalyzed hydrolysis than those in common use mentioned above. The lower rate of hydrolysis means that less choline oxidase, a costly coupling enzyme employed in the assay of cholinesterase activity, is needed. It also means that a more accurate determination of cholinesterase activity can be achieved.

The substrates of the invention are arylcholine esters of the formula: 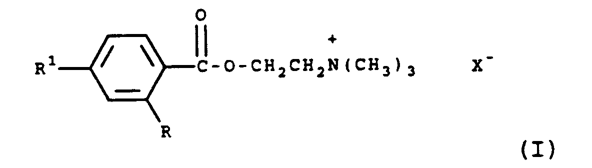
wherein R and R¹ can be the same or different, and can be alkyl, such as methyl, ethyl, propyl, butyl, isopropyl, and so forth; alkoxy, such as methoxy, ethoxy, propoxy, butoxy, and so forth; amino (-NR²R³), wherein R² and R³ can be the same or different, and can be hydrogen, methyl, ethyl, or propyl, wherein X⁻ is an anion, such as halogen (chloro, bromo, or iodo) methyl, sulfate, nitrate and acetate.

Experiments conducted in our laboratory showed that cholinesterase activity toward 2,6- and 2,4,6-substituted benzoylcholines was either absent or too low to be useful for our purposes. Similar results have been reported by J. Thomas and J.R. Stoker, *The Effect of Ortho Substitution in the Hydrolysis of Benzoylcholine,* 13 Journal of Pharmacy and Pharmacology 129-138, (1961).

Thus, it appears that di-substitution is not sufficient to give an advantage as a cholinesterase substrate. Unexpected results are observed with substitution in the ortho and para positions. Especially preferred in this family of compounds are: (1) 2-methyl-4-methoxybenzoylcholine; (2) 2,4-dimethylbenzoylcholine; and (3) 2,4-dimethoxybenzoylcholine. These compounds are of the formula described above with substituents as follow:

**Table I**

| Substrate | R | R¹ | X |
|---|---|---|---|
| 1 | -CH₃ | -OCH₃ | I,C1 |
| 2 | -CH₃ | -CH₃ | I |
| 3 | -OCH₃ | -OCH₃ | I |

Various monosubstituted choline substrates are also known, for example, o-methylbenzoylcholine (o-toluoylcholine), o-methoxybenzoylcholine (o-anisoylcholine), and o-propoxycholine, and so forth However, as shown by the data in Table III, the substrates of the present invention are better, that is, they have slower cholinesterase-catalyzed activities, than the mono-substituted substrates.

The substrates of the invention are prepared by the following general scheme:

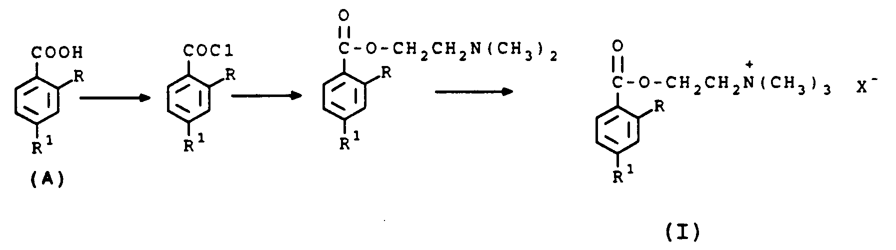

In some cases, the disubstituted benzoic acid starting material (A) is commercially available; otherwise, it can be synthesized as described in Example 1. The preparation of 2-methyl-4-methoxybuzoylcholine iodide, described in Example 1, is typical of the method used to synthesize the other 2,4 di-substituted benzoylcholines of the invention.

The chemical basis for the cholinesterase assay of the present invention is that the substrate is hydrolyzed by cholinesterase in the test sample, liberating choline. The choline is oxidized by the action of choline oxidase to betaine liberating two moles of hydrogen peroxide for each mole of choline. Aminoantipyrine and phenol are then oxidatively coupled by two moles of peroxide catalyzed by the peroxidase resulting in a quinoneimine-type pigment. Any peroxidatively coupled dye would be useful as a color-developing reagent. The rate of increase of color is monitored by reflection densitometry and related to the cholinesterase activity in the sample. The assay is based on published procedures; see, for example, US-A-4,271,310.

The reaction path is: 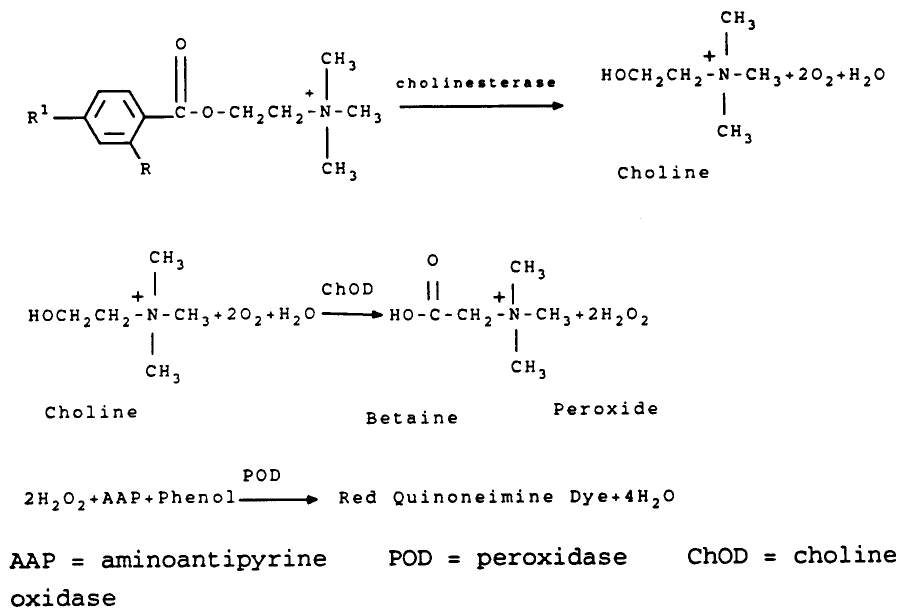
AAP = aminoantipyrine POD = peroxidase ChOD = choline oxidase

The above-described series of chemical reactions can be carried out in solution as described in Example 2 below. Preferably, they are carried out on an automatic analyzer, such as the EKTACHEM™ E700 analyzer, using a dry analytical element of the invention spotted with a sample to be assayed for cholinesterase activity. Dry analytical elements useful for the assay of liquids can be prepared according to the teachings of US-A-3,992,158 and US-A-4,357,363.

Briefly described, the analytical element of this invention comprises a spreading layer and one or more layers coated on a suitable support. All the reagents may be in a single layer coated on the support. Preferably, the reagents are coated in two distinct reagent layers as shown in Table II below. Whether contained in the same or in different layers of the element, all reagents must be in fluid contact with each other, meaning that reagents and reaction products can pass within a layer and between superposed regions of adjacent layers.

The support can be any suitable dimensionally stable, and preferably, nonporous and transparent (that is, radiation transmissive) material which transmits electromagnetic radiation of a wavelength between 200 and 900 nm. A radiation-transmissive support is particularly preferred to enhance and facilitate determination of detectable changes occurring in these elements by use of various radiation detection methods. A support of choice for a particular element should be compatible with the intended mode of detection (reflection, transmission or fluorescence spectroscopy). Useful support materials include polystyrene, polyesters [for example, poly(ethylene terephthalate)], polycarbonates, cellulose esters (for example, cellulose acetate), and so forth.

At least one reagent layer is coated on the support. The reagent layer(s) contain the indicator composition comprising one or more reagents dispersed in one or more synthetic or natural binder materials, such as gelatin or other naturally-occurring colloids, as well as different synthetic hydrophilic polymers such as poly(acrylamide), poly(vinylpyrrolidone), poly(acrylamide-co-N-vinyl-2-pyrrolidone), copolymers of the above, and polymers or copolymers to which crosslinkable monomers have been added.

The reagent layer may contain a buffer. Useful buffers include phosphate, pyrophosphate, tris(hydroxymethyl)aminomethane (TRIS), 2{[tris(hydroxymethyl)methyl)amino}-1-ethanesulfonic acid (TES) and other buffers with pH in the range of 7.0 to 8.5. The buffer may be in any or all layers of the element, or it may be in a separate layer devoid of other reagents.

Several surfactants such as Olin-10G™, TX-405™, Zonyl FSN™ (a family of octylphenoxy polyethoxy ethanol nonionic surfactants sold by Rohm and Haas), and so forth may optionally be included in the reagent layer. Several different cross-linking agents are also optional, such as bisvinylsulfonylmethane, gluteraldehyde, and so forth.

The element is provided with a porous, reflective spreading layer to uniformly distribute the liquid test sample over the element. The spreading layer may contain the reagents, but preferably it is a distinct layer as shown in Table II below. Materials for use in spreading layers are well known in the art of making dry analytical elements as disclosed, for example, in US-A-4,258,001 and the above cited patents.

An exemplary spreading layer is presented in Table II below. Pigments other than barium sulfate could be used, for example, titanium dioxide. Binders other than cellulose acetate may be used, for example, various polyurethanes and other polymers. Surfactants other than TX-405™ may be used, for example, TX-100™.

Other optional layers, for example, subbing layers, radiation-blocking layers, and so forth can be included if desired. The layers of the element can contain a variety of other desirable but optional components, including surfactants, thickeners, buffers, hardeners, antioxidants, coupler solvents, and other materials known in the art. The amounts of these components are also within the skill of a worker in the art.

Changes in the element can be detected with suitable spectrophotometric apparatus, usually a reflectometer, using generally known procedures disclosed, for example, in US-A-3,992,158 at Cols. 14-15 and US-A-4,357,363 at Cols. 27. In an enzymatic reaction, the resulting product is determined by measuring, for example, the rate of change of reflection or transmission density in a finite area of the element of the invention contacted with the assay sample. The area measured is generally from 3 to 5 mm.

A representative element of this invention is presented in Table II below. It will be understood by those skilled in the art that the principle of the present invention can be usefully incorporated into any analytical element employing the method provided by the present case. It will also be understood that cholinesterase activity in other samples besides sera can also be assayed using this element.

**TABLE II**

| An Element of the Invention | | | |
|---|---|---|---|
| | | G/m² | Useful Range G/m² |
| Spreading Layer | Titanium Dioxide | 50.3 | 10-200 |
| | Cellulose Acetate | 398.3 | 50-600 |
| | TX-405 | 1.66 | 0- 20 |
| | Brij 78 | 0.83 | 0- 20 |
| Subbing Layer | poly-N-isopropyl acrylamide | 0.4 | 0.05-5 |
| Reagent Layer | Gelatin | 6.5 | 2-15 |
| | TX-100 | 0.5 | 0.01-2 |
| | TRIS | 1 | 0.5 -5 |
| | Choline Oxidase 2-methyl-4-methoxy benzoylcholine | 2500u/m² | 1000-15,000u/m² |
| | Iodide | 0.5 | 0.2-5 |
| | Bisvinylsulfonylmethylether | 0.05 | 0.005-1.0 |
| Reagent Layer | Gelatin | 6.5 | 2-15 |
| | TX-100 | 0.5 | .01-2 |
| | Alkanol,XC | 0.5 | .01-2 |
| | KS-52 | 3.2 | 0.5-5 |
| | Leuco Dye | 0.32 | 0.05-0.5 |
| | Dimedone | 0.09 | 0-1.0 |
| | TRIS | 1.0 | 0.5-5 |
| | Peroxidase | 32,200u/m² | 5000-50,000u/m² |
| | Bisvinylsulfonylmethylether | 0.05 | 0.005-1.0 |
| u=unit of activity. In the case of choline oxidase, 1 unit causes the formation of 1 micromole of hydrogen peroxide at 37°C, at pH 8.0. In the case of peroxidase, one unit will form 1mG purpurogallin from pyrogallol in 20 seconds at pH 6.0 at 20°C. | | | |

The names and symbols used in the above element and text have the following meanings:
- Brij: polyoxyethylene alcohol
- TX-100™: A family of octylphenoxy polyethoxy
- TX-405™: ethanol nonionic surfactants sold byRohm and Haas
- TRIS: tris(hydroxymethyl)amino-methane
- KS-52: 2,4-di-N-pentylphenol
- Leuco Dye: 2-(3,5-dimethoxy-4-hydroxyphenyl)-4,5-bis(p-dimethylaminophenyl)imidazole

The reagent layer was coated on a subbed, gelatin washed poly(ethylene terephthalate) support, and the spreading layer was coated over the reagent layer. All of the aforementioned layers were coated using conventional coating techniques known in the art for making dry assay elements mentioned above such as those disclosed in US-A-4,357,363 and US-A-3,992,158.

The element of the invention is intended for use with serum or plasma samples but any fluid that sufficiently mimics serum could also be used. These include typical control and proficiency fluids.

No special sample preparation is required other than the separation of serum or plasma from whole blood. Dilution is not required, but is allowed provided the diluted activity is sufficient to be read. The samples can be frozen and maintain activity for long periods of time. They should be thawed and allowed to reach room temperature prior to assay.

The assay of the invention utilizes between 5 and 20 mL of sample. Although the invention allows for variations in the volume of applied samples, 11 mL is preferred. Different calibration curves are required for the different sample volumes. Physical contact is made between the liquid sample under analysis and the analytical element. Such contact can be accomplished in any suitable manner, preferably by spotting the element by hand or machine with a drop of the sample on the spreading layer with a suitable dispensing means.

After sample application, the element is exposed to any conditioning, such as incubation heating or the like, that may be desirable to quicken or otherwise facilitate chemical reaction. Such conditioning is preferably performed at 25-40°C, most preferably at 37°C. The element is monitored for any change in reflectance density during the period of conditioning, usually for 3 to 7 minutes, preferably for 5 minutes, although changes may occur as soon as 20 to 25 seconds, or as long as 30 to 60 minutes.

The preparation of the compounds and the method of determining cholinesterase activity are illustrated by the examples below. The analytical element used in all the examples is that shown in Table II herein.

### EXAMPLE 1 - Preparation of 2-Methyl-4-methoxybenzoylcholine Iodide (Compound 1)

### Step 1: Preparation of 4-Iodo-3-methylanisole (Intermediate A)

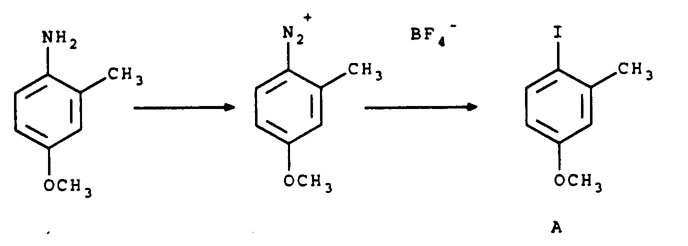

Fourteen grams of 4-methoxy-2-methylaniline (Aldrich) was added neat to a stirred slurry of ice and concentrated hydrochloric acid (30 mL). A large portion of the amine hydrochloride crystallized out. Sodium nitrite solution (8 g in 50 mL of water) was slowly added to the stirred slurry. After the addition, the mixture was filtered, and the filtrate was transferred to a reaction flask. Fluoroboric acid (15 mL of a 49% solution) was added and the mixture was chilled in ice. The tetrafluoroborate salt of the diazonium compound crystallized out.

The solid was collected by filtration and while still damp was dissolved in acetone (∼50 mL). A solution of sodium iodide (16 g in 25 mL of water) was added in portions to the stirred diazonium solution. After the addition, the solution was heated to boiling until nitrogen evolution ceased (about 5-10 minutes). The acetone was removed on a rotary evaporator. Water (about 300 mL) was added, and the mixture was extracted with ether (3 times 200 mL). The combined ether extracts were washed 3 times with dilute sodium bisulfite solution to remove iodine, and the solvent was removed on a rotary evaporator. A thin layer chromatograph on a silica plate developed with hexane showed one major product. The material was crystallized from heptane (about 100 mL) to give 10 g of yellow crystals of A. Structure determination was done by nuclear magnetic resonance (NMR).

### Step 2: Preparation of 4-Methoxy-2-methylbenzoic Acid (Intermediate B)

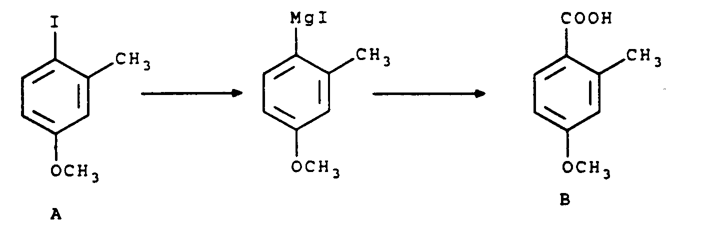

A solution of intermediate A (39 g) in anhydrous diethyl ether (100 mL) was added dropwise to a mixture of magnesium metal (4 g) in anhydrous diethylether (about 200 mL), observing the usual precautions on running a Grignard reaction. After the addition, the reaction mixture was heated under reflux for one hour.

The Grignard solution was poured into a slurry of dry ice (large excess) and diethylether (about 200 mL) and allowed to stand until the ice melted and the mixture warmed to room temperature. Dilute hydrochloric acid (about 200 mL) was added cautiously. The mixture was shaken by hand to mix and then filtered through a sintered glass funnel. The solid on the funnel was washed alternately with ether and dilute hydrochloric acid until nearly all of the solid had been dissolved and gone through the filter. The filtrate was warmed to redissolve the solids, the water layer was separated, and the ether layer was washed with dilute hydrochloric acid (200 mL). The ether was removed on a rotary evaporator, and the residue was extracted with dilute sodium bicarbonate solution (3 times 200 mL). The sodium bicarbonate solution was extracted with diethylether (100 mL) to remove insoluble impurities. The water layer was then made acidic with concentrate hydrochloric acid, and the product was collected and washed well with water to give 10.5 g of intermediate B. A thin layer chromatograph on a silica plate developed with toluene, ethyl acetate, and acetic acid (8,2,1) showed one major product. Structure determination was done by nuclear magnetic resonance.

### Step 3: Preparation of the Choline Ester (Intermediate C)

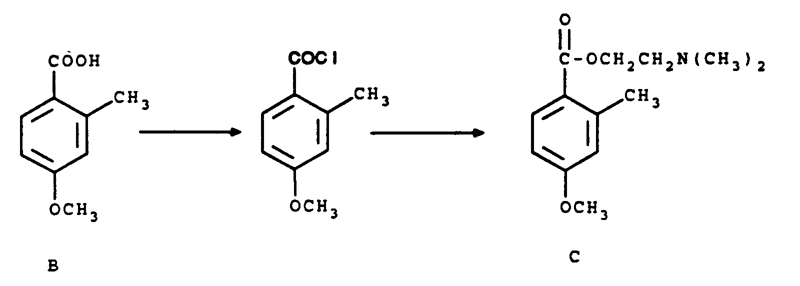

Solid intermediate B (10 g) was added in small portions to a stirred solution of oxalyl chloride (100 g). After the addition, the solution was heated under reflux for 30 minutes, then concentrated on a rotary evaporator. The residue was dissolved in acetonitrile (100 mL) and added dropwise to a stirred, cooled solution of dimethylaminoethanol (20 g) in acetonitrile (150 mL). After the addition, the solution was allowed to warm to room temperature and then stirred for one hour. The acetonitrile was removed on a rotary evaporator, and the residue was treated with water (100 mL). The aqueous mixture was extracted with ether (3 times 100 mL), and the ether solution was washed with cold water (100 mL) several times. The ether solution was dried (magnesium sulfate) and used directly in the next step. A thin layer chromatograph on a silica plate developed with ethylacetate/methanol (9/1) showed one major spot.

### Step 4: Preparation of 2-Methyl-4-methoxybenzoylcholine Iodide (Compound 1)

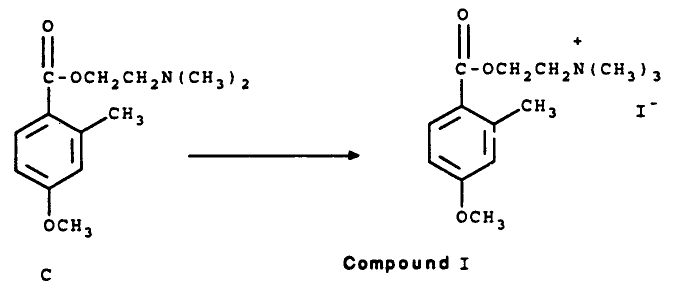

The ester solution from step 3 containing intermediate C was treated with iodomethane (8 g). The resulting solution was allowed to stand in a stoppered flask overnight at room temperature. The white solid product was collected by filtration and washed well with dry ether to give 18 g of compound 1. A thin layer chromatograph on a silica plate developed with ethylacetate/methanol/acetic acid (2,2,1) showed one major spot. Structure was confirmed by nuclear magnetic resonance.

### EXAMPLE 2 - Known Substrates Compared With Substrates of the Invention in a Solution Assay

The substrates of the present invention and of the prior art were tested in solution as shown in Table II:

**TABLE III**

| | |
|---|---|
| TRIS buffer, 8.0 | 200 mM |
| Phenol | 30 mM |
| 4-Aminoantipyrine | 0.8 mM |
| Peroxidase | 90 U/mL |
| Choline oxidase | 11.2 U/mL |
| Choline esterase | 0.22 U/mL |
| Substrates | 0.01-50 mM |

The substrates of this invention and of the prior art were tested in solution using the composition shown in Table III. The test was run on a Cobas Fara II centrifugal fast analyzer at 37°C for a six minute incubation period. Absorbance readings were taken at 500 nm over 10 second intervals. The rate of absorbance change, DA/min, was calculated and related to the cholinesterase activity. Assayed in this manner, the relative rates of activity with the new substrates is shown in Table IV.

**TABLE IV**

| Test Results | |
|---|---|
| Substrate | Relative Activities (1 mmolar substrate, pH 8.0) |
| Control (o-Tolylcholine chloride) | 18 |
| Control (o-Propoxycholine chloride) | 40 |
| 1 | 3 |
| 2 | 8 |
| 3 | 12 |

The data indicate that 2,4-disubstituted substrates have desirably lower enzymatic rates of hydrolysis than the mono-ortho-substituted compounds.

### EXAMPLE 3 - Analytical Element with Coating of 2-Methyl-4-Methoxybenzoyl Choline as Substrate

An element was made as shown in Table II and tested for its performance on an EKTACHEM™ analyzer. Water and six fluids with varying cholinesterase levels (48, 359, 650, 946, 1504, and 1895 U/L) were spotted on the element and assayed for cholinesterase activity according to the method described by Panteghini and Bonora (J. Clin. Chem. Clin. Biochem., 22, 1984, p.671-6), modified in that we replaced benzoylcholine in the reference with equal amounts of o-toluoylcholine. The reading was done at 670 nm.

The results are shown in Figure 1 which is a plot of the kinetics, reflectance density versus time. The plot shows 7 distinct slopes corresponding to the 7 fluids tested. This demonstrates the broad dynamic range and accuracy of the element of the invention.

## Claims

1. A compound of the formula: 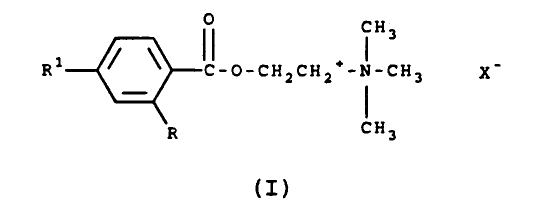 wherein R and R¹ each independently represents alkyl, alkoxy, amino (-NR²R³), wherein R² and R³ each independently represents hydrogen, methyl, ethyl, or propyl, and X⁻ is an anion.

2. The compound as claimed in claim 1 wherein alkyl is methyl, ethyl, propyl, butyl, or isopropyl.

3. The compound as claimed in claim 1 wherein alkoxy is methoxy, ethoxy, propoxy,or butoxy.

4. The compound as claimed in claim 1 wherein X⁻ is chloro-, bromo-, iodo-, methyl, sulfate, nitrate or acetate.

5. A reagent composition for determining cholinesterase activity in an aqueous sample comprising, in combination, a substrate for serum cholinesterase, choline oxidase, peroxidase, a color-developing reagent, and a buffer having a pH of 7 to 9 characterized in that the substrate for serum cholinesterase is a compound of the formula: 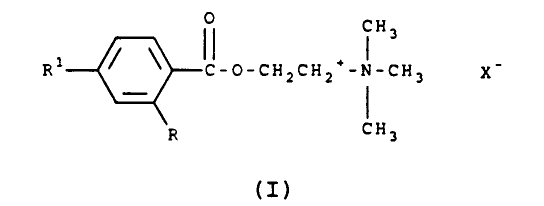 wherein R and R¹ each independently represents alkyl, alkoxy, amino (-NR²R³), wherein R² and R³ each independently represents hydrogen, methyl, ethyl, or propyl, and X⁻ is an anion.

6. A multilayer analytical element for the determination of serum cholinesterase comprising a support having thereon, in order from the support and in fluid contact, at least one reagent layer and a spreading layer, and containing in the reagent layer:
a) a substrate for serum cholinesterase;
b) choline oxidase;
c) peroxidase;
d) a color-developing reagent; and
e) a buffer having a pH of 7 to 9
characterized in that the substrate for serum cholesterase is a compound of the formula: 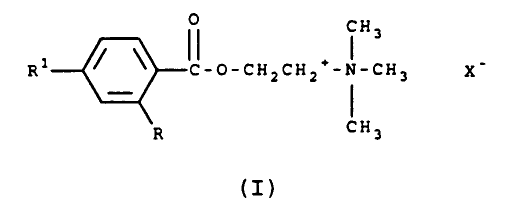 wherein R and R¹ each independently represents alkyl, alkoxy, amino (-NR²R³), wherein R² and R³ each independently represents hydrogen, methyl, ethyl, or propyl, and X⁻ is an anion.

7. The analytical element as claimed in claim 6 comprising:
a first reagent layer containing a binder material, peroxidase, choline oxidase, and a color-developing reagent which provides a detectable change in response to the reaction of the substrate with serum cholinesterase;
a second reagent layer containing the substrate;
a buffer having a pH of 7 to 9 in either or both reagent layers; and
an isotropically porous spreading layer.

8. A multilayer analytical element for the determination of serum cholinesterase, the element comprising a support having thereon, in order from the support and in fluid contact:
a first reagent layer containing gelatin, a buffer having a pH of 7 to 9, peroxidase, and aminoantipyrine coupled with phenol;
a second reagent layer containing gelatin, choline oxidase, a buffer having a pH of 7 to 9, and a substrate for serum cholinesterase; and
an isotropically porous spreading layer characterized in that the substrate for serum cholinesterase is selected from the group consisting of 2,4-dimethylbenzoylcholine iodide, 2-methyl-4-methoxybenzoylcholine iodide, and 2,4-dimethoxybenzoylcholine iodide.

9. A method for determining serum cholinesterase in an aqueous liquid comprising the steps of:
a) contacting a sample of the aqueous liquid with a multilayer analytical element comprising a support having thereon in order from the support and in fluid contact, a spreading layer and at least one reagent layer and containing in the reagent layer:
a substrate for serum cholinesterase;
choline oxidase;
peroxidase;
a buffer having a pH of 7 to 9; and
a color-developing agent which provides a detectable change in response to the reaction of the substrate with serum cholinesterase; and
b) determining the cholinesterase activity in the sample by means of the detectable change thereby obtained; characterized in that the substrate for serum cholinesterase is of the formula: 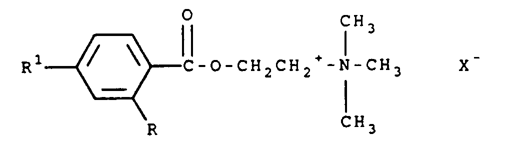 wherein R and R¹ each independently represents alkyl, alkoxy, amino (-NR²R³), wherein R² and R³ each independently represents hydrogen, methyl, ethyl, or propyl, and X⁻ is an anion.

10. The method as claimed in claim 9 wherein the detectable change is an optical density change.

## Patentansprüche

1. Verbindung der Formel 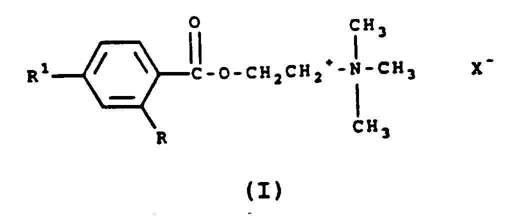 in der R und R¹ jeweils unabhängig voneinander Alkyl, Alkoxy oder Amino (-NR²R³), bedeuten, worin R² und R³ jeweils unabhängig voneinander Wasserstoff, Methyl, Ethyl oder Propyl bedeuten, und X⁻ ein Anion bedeutet.

2. Verbindung nach Anspruch 1, wobei Alkyl die Bedeutung Methyl, Ethyl, Propyl, Butyl oder Isopropyl hat.

3. Verbindung nach Anspruch 1, wobei Alkoxy die Bedeutung Methoxy, Ethoxy, Propoxy oder Butoxy hat.

4. Verbindung nach Anspruch 1, wobei X⁻ Chlor, Brom, Iod, Methyl, Sulfat, Nitrat oder Acetat bedeutet.

5. Reagenzzusammensetzung zur Bestimmung der Cholinesterase-Aktivität in einer wäßrigen Probe, umfassend in Kombination ein Substrat für Serum-Cholinesterase, Cholinoxidase, Peroxidase, ein Farbentwicklungsmittel und einen Puffer mit einem pH-Wert von 7 bis 9, dadurch gekennzeichnet, daß es sich bei dem Substrat für Serum-Cholinesterase um eine Verbindung der folgenden Formel handelt 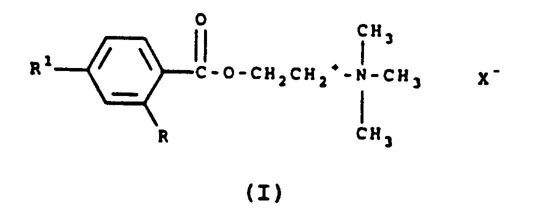 in der R und R¹ jeweils unabhängig voneinander Alkyl, Alkoxy oder Amino (-NR²R³), bedeuten, worin R² und R³ jeweils unabhängig voneinander Wasserstoff, Methyl, Ethyl oder Propyl bedeuten, und X⁻ ein Anion bedeutet.

6. Mehrschichtiges analytisches Element zur Bestimmung von Serum-Cholinesterase, umfassend einen Träger, auf dem sich nacheinander ausgehend vom Träger und in Fluidkontakt mindestens eine Reagenzschicht und eine Verteilungsschicht befinden, wobei in der Reagenzschicht folgende Bestandteile enthalten sind:
a) ein Substrat für Serum-Cholinesterase;
b) Cholin-oxidase;
c) Peroxidase;
d) ein Farbentwicklungsmittel; und
e) ein Puffer mit einem pH-Wert von 7 bis 9,
dadurch gekennzeichnet, daß es sich bei dem Substrat für Serum-Cholinesterase um eine Verbindung der folgenden Formel handelt: 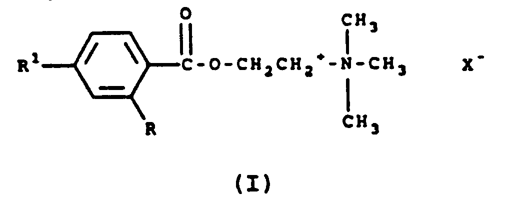 in der R und R¹ jeweils unabhängig voneinander Alkyl, Alkoxy oder Amino (-NR²R³), bedeuten, worin R² und R³ jeweils unabhängig voneinander Wasserstoff, Methyl, Ethyl oder Propyl bedeuten, und X⁻ ein Anion bedeutet.

7. Analytisches Element nach Anspruch 6, umfassend
eine erste Reagenzschicht mit einem Gehalt an einem Bindemittelmaterial, Peroxidase, Cholin-oxidase und einem Farbentwicklungsmittel, das eine nachweisbare Veränderung als Antwort auf die Reaktion des Substrats mit Serum-Cholinesterase liefert;
eine zweite Reagenzschicht mit einem Gehalt an dem Substrat;
einen Puffer mit einem pH-Wert von 7 bis 9 in einer oder beiden Reagenzschichten; und
eine isotrop poröse Verteilungsschicht.

8. Mehrschichtiges analytisches Element zur Bestimmung von Serum-Cholinesterase, wobei das Element einen Träger umfaßt, auf dem in der angegebenen Reihenfolge und in Fluidkontakt folgende Bestandteile angeordnet sind:
eine erste Reagenzschicht mit einem Gehalt an Gelatine, einem Puffer mit einem pH-Wert von 7 bis 9, Peroxidase und mit Phenol gekuppeltem Aminoantipyrin;
eine zweite Reagenzschicht mit einem Gehalt an Gelatine, Cholin-oxidase, einem Puffer mit einem pH-Wert von 7 bis 9 und einem Substrat für Serum-Cholinesterase; und
eine isotrop poröse Verteilungsschicht,
dadurch gekennzeichnet, daß das Substrat für Serum-Cholinesterase aus der Gruppe 2,4-Dimethylbenzoylcholiniodid, 2-Methyl-4-methoxybenzoylcholiniodid und 2,4-Dimethoxybenzoylcholiniodid ausgewählt ist.

9. Verfahren zur Bestimmung von Serum-Cholinesterase in einer wäßrigen Flüssigkeit, umfassend folgende Stufen:
a) Kontaktieren einer Probe der wäßrigen Flüssigkeit mit einem mehrschichtigen analytischen Element, das einen Träger umfaßt, auf dem in der angegebenen Reihenfolge ausgehend vom Träger und in Fluidkontakt eine Verteilungsschicht und mindestens eine Reagenzschicht angeordnet sind, wobei die Reagenzschicht folgende Bestandteile enthält:
ein Substrat für Serum-Cholinesterase;
Cholin-oxidase;
Peroxidase;
einen Puffer mit einem pH-Wert von 7 bis 9; und
ein Farbentwicklungsmittel, das eine nachweisbare Veränderung in Antwort auf die Reaktion des Substrats mit Serum-Cholinesterase liefert; und
b) Bestimmen der Cholinesterase-Aktivität in der Probe mittels der dabei erhaltenen nachweisbaren Veränderung, dadurch gekennzeichnet, daß das Substrat für Serum-Cholinesterase folgende Formel aufweist 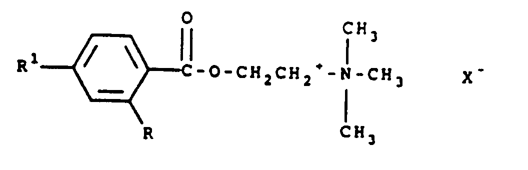 in der R und R¹ jeweils unabhängig voneinander Alkyl, Alkoxy oder Amino (-NR²R³), bedeuten, worin R² und R³ jeweils unabhängig voneinander Wasserstoff, Methyl, Ethyl oder Propyl bedeuten, und X⁻ ein Anion bedeutet.

10. Verfahren nach Anspruch 9, wobei es sich bei der nachweisbaren Änderung um eine Änderung der optischen Dichte handelt.

## Revendications

1. Composé de formule: 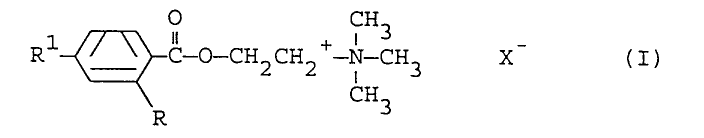 dans laquelle R et R¹ représentent chacun indépendamment un groupe alkyle, alcoxy, amino (-NR²R³) dans lequel R² et R³ représentent chacun indépendamment un atome d'hydrogène ou le radical méthyle, éthyle ou propyle, et X⁻ est un anion.

2. Composé selon la revendication 1, dans lequel le groupe alkyle est le groupe méthyle, éthyle, propyle, butyle ou isopropyle.

3. Composé selon la revendication 1, dans lequel le groupe alcoxy est le groupe méthoxy, éthoxy, propoxy ou butoxy.

4. Composé selon la revendication 1, dans lequel X⁻ est l'anion chloro-, bromo-, iodo-, méthyle, sulfate, nitrate ou acétate.

5. Composition de réactif pour la détermination de l'activité cholinestérase dans un échantillon aqueux, comprenant, en association, un substrat pour la cholinestérase sérique, de la choline oxydase, un peroxydase, un réactif chromogène et un tampon ayant un pH de 7 à 9, caractérisée en ce que le substrat pour la cholinestérase sérique est un composé de formule: 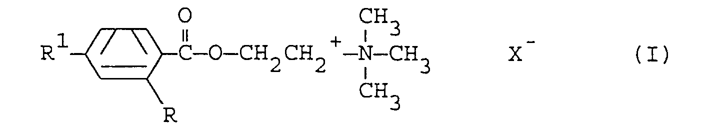 dans laquelle R et R¹ représentent chacun indépendamment un groupe alkyle, alcoxy, amino (-NR²R³) dans lequel R² et R³ représentent chacun indépendamment un atome d'hydrogène ou le radical méthyle, éthyle ou propyle, et X⁻ est un anion.

6. Elément analytique multicouche pour la détermination de la cholinestérase sérique, comprenant un support sur lequel se trouve, dans l'ordre à partir du support et en contact fluide, au moins une couche de réactif et une couche d'étalement, et contenant dans la couche de réactif:
a) un substrat pour la cholinestérase sérique;
b) de la choline oxydase;
c) de la peroxydase;
d) un réactif chromogène; et
e) un tampon ayant un pH de 7 à 9,
caractérisé en ce que le substrat pour la cholestérase sérique est un composé de formule: 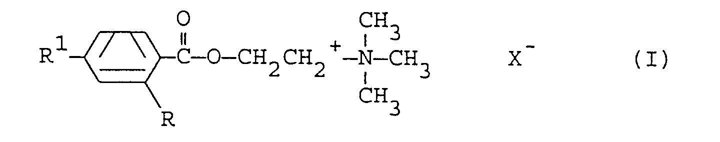 dans laquelle R et R¹ représentent chacun indépendamment un groupe alkyle, alcoxy, amino (-NR²R³) dans lequel R² et R³ représentent chacun indépendamment un atome d'hydrogène ou le radical méthyle, éthyle ou propyle, et X⁻ est un anion.

7. Elément analytique selon la revendication 6, comprenant:
une première couche de réactif contenant une substance jouant le rôle de liant, de la peroxydase, de la choline oxydase et un réactif chromogène produisant un changement détectable en réponse à la réaction du substrat avec la cholinestérase sérique;
une seconde couche de réactif contenant le substrat;
un tampon ayant un pH de 7 à 9 dans l'une ou l'autre couche de réactif ou les deux; et
une couche d'étalement isotropiquement poreuse.

8. Elément analytique multicouche pour la détermination de la cholinestérase sérique, l'élément comprenant un support sur lequel se trouvent, dans l'ordre à partir du support et en contact fluide:
une première couche de réactif contenant de la gélatine, un tampon ayant un pH de 7 à 9, de la peroxydase et de l'aminoantipyrine couplée au phénol;
une seconde couche de réactif contenant de la gélatine, de la choline oxydase, un tampon ayant un pH de 7 à 9 et un substrat pour la cholinestérase sérique; et
une couche d'étalement isotropiquement poreuse, caractérisé en ce que le substrat pour la cholinestérase sérique est choisi parmi l'iodure de 2,4-diméthylbenzoylcholine, l'iodure de 2-méthyl-4-méthoxybenzoylcholine et l'iodure de 2,4-diméthoxybenzoylcholine.

9. Procédé pour la détermination de la cholinestérase sérique dans un liquide aqueux, comprenant les étapes suivantes:
a) mise en contact d'un échantillon du liquide aqueux avec un élément analytique multicouche comprenant un support sur lequel se trouve, dans l'ordre à partir du support et en contact fluide, une couche d'étalement et au moins une couche de réactif, et contenant dans la couche de réactif:
un support pour la cholinestérase sérique;
de la choline oxydase;
de la peroxydase;
un tampon ayant un pH de 7 à 9; et
un agent chromogène produisant un changement détectable, en réponse à la réaction du substrat avec la cholinestérase sérique; et
b) la détermination de l'activité cholinestérase dans l'échantillon au moyen du changement détectable ainsi obtenu;
caractérisé en ce que le substrat pour la cholinestérase sérique est de formule: 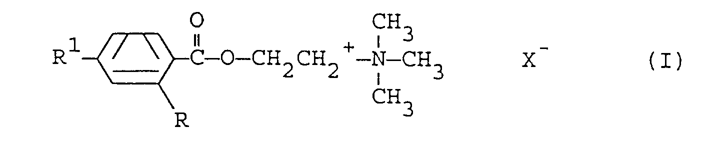 dans laquelle R et R¹ représentent chacun indépendamment un groupe alkyle, alcoxy, amino (-NR²R³) dans lequel R² et R³ représentent chacun indépendamment un atome d'hydrogène ou le radical méthyle, éthyle ou propyle, et X⁻ est un anion.

10. Procédé selon la revendication 9, dans lequel le changement détectable est un changement de densité optique.
